# EUROPEAN PATENT APPLICATION

(11) **EP 0 998 957 A2**
(43) Date of publication of application: **10.05.2000**
(21) Application number: 99122116.9
(22) Date of filing: 05.11.1999
(51) Int. Cl.: A61N 1/05

(54) **Cardiac electrode having improved stability**

(30) Priority: 05.11.1998 IL 12690498
(71) Applicant: IMPULSE DYNAMICS N.V., Curacao (AN)
(72) Inventor: Malonek, Dov, 36092 Tivon (IL)
(74) Representative: Gervasi, Gemma, Dr.

(57) **Abstract**

An improved cardiac catheter having at least one alarm conductor (16) that is not insulated, which comprises electrodes, a catheter stem (15), having an insulating sleeve, and insulated functional conductors (13) that electrically connect the electrodes (17) with a control unit (18'), and at least an alarm conductor (16, 22) that is not insulated.

The alarm conductor (16) may be not one of the functional conductors (13) of the catheter, or a functional conductor (13) of the catheter, which is not insulated and has the additional function of providing an alarm signal (19). The alarm conductor (16) may be connected in parallel to a functional conductor (13).

## Description

### Field of the Invention

The invention relates to improvements in the functional safety of cardiac catheters, and, more particularly, to a structure of the catheter's stem whereby warning is given of possible future catheter failure.

### Background of the Invention

Cardiac catheters are used for purposes such as mapping, pacing, and defibrillation are well known in the art. The use of cardiac catheters to apply a non-stimulatory signal to the cardiac muscle tissue has been recently described (see, for instance, PCT Patent Application PCT/IL97/00012, published as WO 97/25098). An improved multi-electrode catheter is described in copending Application, attorney's docket No. 4965/98, the contents of which are incorporated herein by reference.

The catheters in question comprise electrodes and electrical conductors which operatively connect the electrodes to a control unit. In the aforesaid applications, electric signals are transmitted from the electrodes to the control unit through conductors, and/or electrical stimulatory or non-stimulatory signals are transmitted from the control unit to the electrodes through conductors. The conductors are generally included in a stem connected as its distal end to the control unit and to sources of power, which may be a part of the control unit. It is essential that the conductors maintain their conductivity for as long as possible. For this purpose, the stem has an insulating cover, and the conductors, too, are preferably provided with insulating coatings or covers. For insulating purposes, such materials as silicone and polyurethanes have been proposed. However, insulation failure often occurs in some cases, resulting in failure of the conductors to deliver the electrical energy to the final target - the cardiac tissue. The electrical energy is delivered through the insulation failure instead of through the electrode. The resulting catheter failure is sudden, with no warning, and there is no known way of guarding against it and its consequences. It is preferred to protect the catheter conductors by a double insulation: an insulating coating or cover on each conductor, and an overall insulation constituted by an insulating sleeve of the catheter's stem.

Corrosion of the insulating sleeve of the catheter's stem often occurs, cracks or gaps form therein, blood penetrates into the stem and comes into contact with the insulation of the several conductors, and a second stage corrosion of the conductors' insulation occurs, finally resulting in the failure of one or more of the conductors and therefore of the entire catheter. The failure of the external sleeve exposes all the coated conductors to body fluids and thus to possible failure, and thus to a failure of the whole catheter. At present the controller may tolerate failure of a single conductor by pacing mode switch, but failure of several conductors may be fatal. Such a failure occurs unpredictably, with serious possibly dangerous consequences. No way of preventing such an occurrence is known in the art. It cannot be prevented by thickening the insulation of the conductors and of the stem, because the catheter must
have a diameter small enough to permit easy introduction into the cardiac veins through which it must pass or in which it must be operative, and thickening the insulation to a significant degree would lead to an unacceptably diameter increase. It is therefore a purpose of this invention to provide an improvement in catheter structure that causes early warnings to be given of conditions leading to catheter failure and permits early intervention, e.g. exchange of the catheter with an undamaged one, before the damaged catheter ceases to function. It is another purpose of the invention to provide a catheter stem which includes a component or components for early warning that a damage of the insulation of the stem sleeve has occurred and that it is likely that the insulation of the individual conductors is being or will be attacked or damaged. It is a further purpose of this invention to achieve the aforesaid purposes without a substantial increase in the diameter of the catheter. It is a still further purpose of this invention to achieve the aforesaid purposes with minimal modification of the catheter structure. It is a still further purpose of this invention to achieve the aforesaid purposes without in any way interfering the normal operation of the catheter.

Other purposes and advantages of the invention will appear as the description proceeds.

### Summary of the Invention

The above mentioned purposes are achieved by mean of a cardiac catheter which, in accordance with main claim 1, comprises electrodes, a catheter stem having an insulating sleeve, and insulated functional conductors that electrically connect the electrodes with a control unit, characterised by providing at least one alarm conductor that is not insulated.

Optionally, a plurality of alarm conductors may be provided. The improved catheter further comprises an alarm unit, which is preferably a part of the control unit, for monitoring the operation of the alarm conductor. If the catheter insulating sleeve fails and ceases to insulate the inside of the catheter completely, blood will seep into the catheter and come into contact with the alarm conductor and, since blood conducts electricity, this will affect the operation of the alarm conductor. The alarm unit will sense a change in the operation of the alarm conductor and generate an alarm. However, the other conductors being provided with their own insulation, they will continue to operate normally for some time after the catheter insulating sleeve has failed and the alarm has been generated. This will provide some time for an intervention to assure continuation of the functions which the catheter carries out, e.g. time for
replacement of the catheter before it fails, and, in any case, for whatever medical intervention may be appropriate to prevent any damage to the patient. The alarm conductor may be independent and not part of a functional circuit of the catheter (viz. a circuit through which a signal is transmitted from the control unit to an electrode, or vice versa) or it may be part of such a circuit, its alarm function being additional, or it may be parallel to a functional conductor. In the first case, the alarm conductor may be connected to the alarm unit at both terminals. The conductor then has two branches, an outgoing one (from one of the terminals to any appropriate position close to the distal end of the catheter) and a parallel incoming one. The two branches should be sufficiently separated, e.g. by a number of insulated conductors, to avoid electrical contact between them. A signal is outputted by the alarm unit to the terminal of the outgoing branch and is monitored by said unit at the terminal of the incoming branch. If blood comes into contact with the alarm conductor, a partial short-circuit between the two branches is produced and the monitored signal is changed, indicating a failure of the insulating sleeve. In the second case, the distal terminal of the alarm conductor may be connected to an electrode of the catheter and the proximate terminal to the control unit, and said connections be maintained, so that a cardiac electric signal is constantly transmitted and can be monitored. Each
signal from a sensing electrode has a shape characterised by rapid changes in voltage that occur every 300 to 1000 msec. In order to monitor the impedance between the conductor and the body fluids, the control unit will send a test signal during the quiet period and then measure the current generated. This signal will be of low amplitude and very short duration, and will thus not interfere with the normal activity of the heart. The control unit monitors the input from all electrodes, and if said test signal changes, while the other functional conductors continue to function normally, this indicates a failure of the insulating sleeve. It may be advantageous to operate in this way, so as not to increase the number of conductors, and therefore the cross-section of the catheter, beyond what is required for functional purposes. In the third case, the alarm conductor may connect the control unit to an electrode, in parallel to a functional conductor. If the signal transmitted by the alarm conductor changes, while that transmitted by the functional conductor does not, a failure of the insulating sleeve is indicated. In a further variant of the third case, an alarm conductor is connected to the alarm unit and runs in parallel to the functional conductors, but it is not connected to an electrode at its distal end. The impedance between the alarm conductor and the external fluids is monitored. An abrupt change in this impedance indicates an insulation failure.

If a plurality of alarm conductors is provided, they could have their distal ends connected to the same sensing electrode or to different sensing electrodes. The alarm unit may be a part of the control unit, may be a separate device, or may be constituted by a number of structurally separate but functionally combined components. Therefore the term "alarm unit", whenever used herein, should be construed as including all the aforesaid possibilities. An alarm generator is included in it or is connected to and activated by it. The alarm may be of any kind, e.g. acoustic or visual or vibratory or a combination thereof, and the structure of the alarm generator will depend on its kind, e.g. will consist of an acoustic alarm generator or a visual display or both. All of these alternatives will be obvious to skilled persons and easily devised by them, and therefore need not be described.

### Brief Description of the Drawings

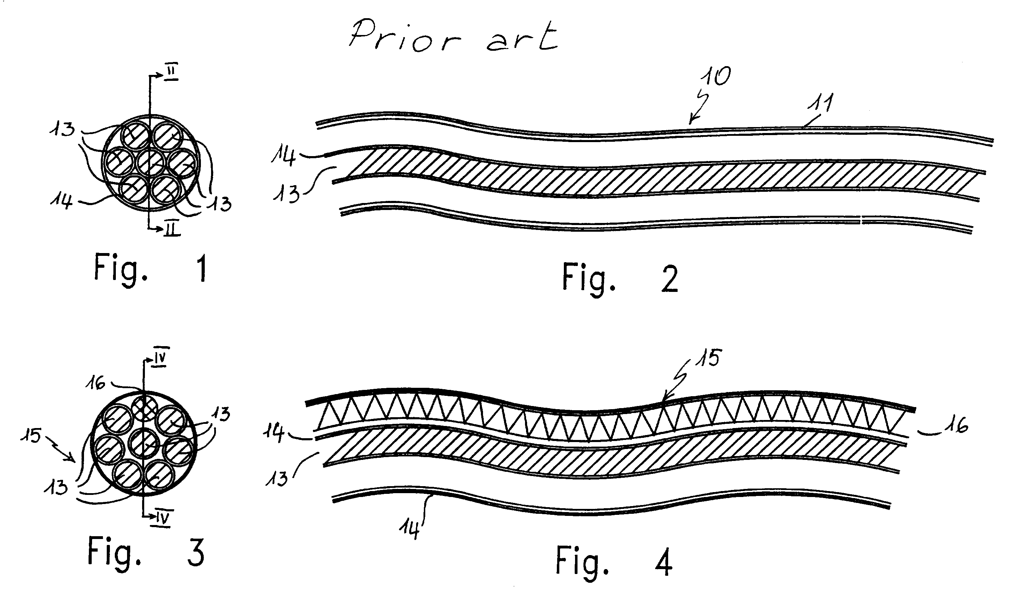

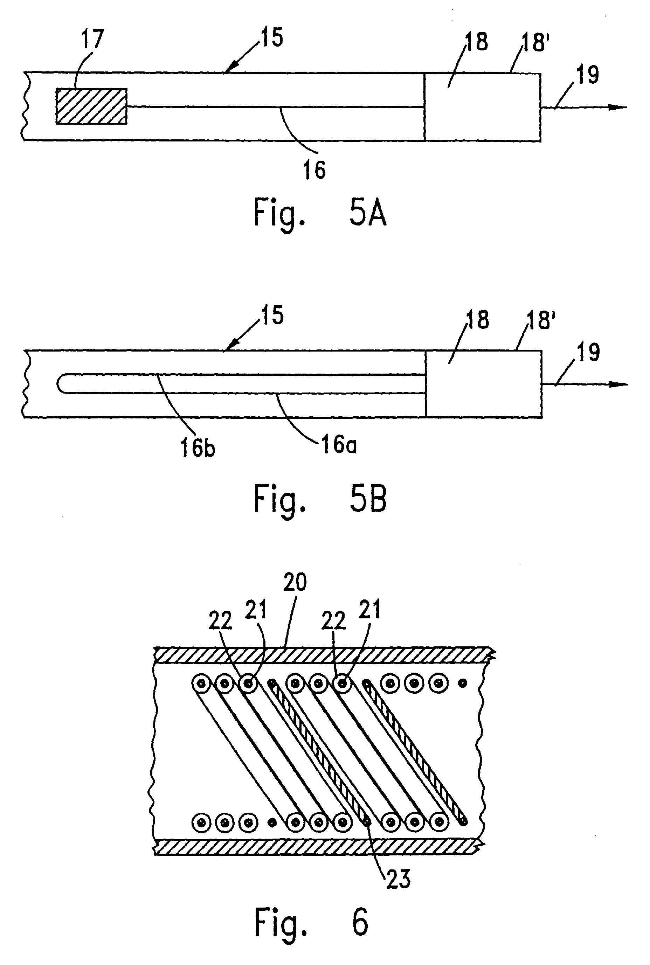

In the drawings:
■ Fig. 1 is a cross-section of a catheter stem according to the prior art;
■ Fig. 2 is an axial cross-section of the stem of Fig. 1, taken on plane II-II of Fig. 1;
■ Fig. 3 is a cross-section similar to Fig. 1, but showing one embodiment of the invention;
■ Fig. 4 is an axial cross-section of the stem of Fig. 3, taken on plane IV-IV of Fig. 3;
■ Figs. 5a and b are block diagrams of an embodiment of the invention; and
■ Fig. 6 illustrates in fragmentary axial cross-section another embodiment of the invention.

### Detailed description of Preferred Embodiments

Figs. 1 and 2 illustrate a conventional stem, globally indicated with reference numeral 10, which has an outer insulation 11 and comprises conductors 13, each of which has an insulation 14. In the figures, the conductors are in the number of six, but this number has no relevance, as different catheters will comprise different numbers of conductors, often in excess of six. The embodiment of the invention illustrated in Figs. 3 and 4 is a stem 15, which comprises the same elements of Figs. 1 and 2, but additionally the alarm conductor 16, which is not insulated (indicated in zig-zag line in Fig. 4). In this embodiment, the alarm conductor has a single branch and is connected at one end to the control unit and at the other end to a sensing electrode, not shown in the drawings. The operation of the device is illustrated by the block diagrams of Fig. 5a and b. In Fig. 5a, alarm conductor 16, included in stem 15, is connected at one terminal to control unit 18' and the other terminal to sensing electrode 17. In Fig. 5b, the alarm conductor comprise an outgoing branch 16a and an incoming branch 16b, and both its terminals are proximate and connected to the alarm unit 18 which can be part of the control unit 18' or separated from it. In the first case, the control unit monitors the cardiac electrical signal coming from an electrode 17 through conductor 16. If blood or other conductive material comes into contact with the alarm conductor, a partial shortcut between the alarm conductor and the external fluid (or medium) is produced, causing a change in the monitored signal. This change in signal causes alarm unit 18 to generate an alarm signal 19, which may be acoustic or visual or both, or, in general, be of any convenient kind. In the second case, the alarm conductor comprises an outgoing branch 16a and an incoming branch 16b, both having a terminal connected to the alarm unit 18. This latter inputs a signal in branch 16a and monitors the signal returning through branch 16b, and if this is changed, generates the alarm signal 19. The alarm conductor is preferably made of MP35N or DFT, of preferably a strand having a diameter of 0,1 mm. Fig. 6 shows another embodiment of the invention, in which the conductors are wound in spiral form. Numeral 20 indicate the insulating sleeve and numeral 21 the conductors having an insulation 22. The non-insulated conductor, provided according to the invention, is indicated at 23 and is hatched in the drawing, though it is not in cross-section, for the purpose of evidencing it. While embodiments of the invention have been described by way of illustration, it will be apparent that many modifications, variations and adaptations may be carried out by persons skilled in the art without departing from its spirit or exceeding the scope of the claims.

## Claims

1. A cardiac catheter which comprises electrodes, a catheter stem having an insulating sleeve, and insulated functional conductors that electrically connect the electrodes with a control unit, characterised by providing at least one alarm conductor (16, 23) that is not insulated.

2. The cardiac catheter of claim 1, wherein the alarm conductor (16, 23) is not one of the functional conductors (13, 21) of the catheter.

3. The cardiac catheter of claim 1, wherein the alarm conductor (16, 23) is a functional conductor (13, 21) of the catheter, which is not insulated and has the additional function of providing an alarm signal (19).

4. The cardiac catheter of claim 1, wherein the alarm conductor (16, 23) is in parallel to a functional conductor (13, 21).

5. The cardiac catheter of claim 1, further comprising an alarm unit (18), operatively connected to the alarm conductor (16, 23) to monitor the signal received through it.

6. The cardiac catheter of claim 5, wherein the alarm unit (18) comprises an alarm generator.

7. The cardiac catheter of claim 5, wherein the alarm unit (18) is operatively connected to an alarm generator.

8. The cardiac catheter of claim 5, wherein the alarm signal (19) is chosen from among acoustic, visual or vibratory alarms or combinations thereof.

9. The cardiac catheter of claim 5, wherein the alarm unit (18) comprises means for causing an alarm (19) to be generated responsive to changes in the signal received from the alarm conductor (16).

10. The cardiac catheter of claim 5, wherein the alarm unit (18) is part of the control unit (18').

11. The cardiac catheter of claim 5, wherein the alarm unit (18) is a separate device from the control unit (18').

12. The cardiac catheter of claim 5, wherein the alarm unit (18) is constituted by a number of structurally separate but functionally combined components.

13. The cardiac catheter of claim 5, wherein the alarm conductor (16) has its distal terminal connected to a sensing electrode (17) of the catheter and its proximate terminal to the alarm unit (18).

14. The cardiac catheter of claim 5, wherein both terminals of the alarm conductor (16) are proximate and connected to the alarm unit (18), said conductor (16) having an outgoing and an incoming branch (16a, 16b).

15. The cardiac catheter of claim 13, wherein the conductors (21, 23) are disposed in spiral form.
